# EUROPEAN PATENT APPLICATION

(11) **EP 4 512 367 A1**
(43) Date of publication of application: **26.02.2025**
(21) Application number: 23192561.1
(22) Date of filing: 22.08.2023
(51) Int. Cl.: A61C 17/02, A61C 19/06

(54) **DENTAL DEVICE WITH INTEGRATED FLUIDIC CHANNELS AND METHOD FOR MANUFACTURING SAID DENTAL DEVICE**

(71) Applicant: CSEM Centre Suisse d'Electronique et de Microtechnique SA, 2002 Neuchâtel (CH)
(72) Inventor: KALENTICS, Nikola, 1007 Lausanne (CH); BOUDOIRE, Florent, 2000 Neuchâtel (CH); PEJCHAL, Vaclav, 1110 Morges (CH); SEREDA, Olha, 2068 Hauterive (CH)
(74) Representative: e-Patent SA

(57) **Abstract**

The present invention is related to a dental device (1) intended to cover at least part of a teeth (2) and optionally at least part of gums (3) or other surfaces of a mouth for repositioning, retaining, protecting and/or treating said teeth (2) and optionally said gums (3) or other surfaces of the mouth, said dental device (1) comprising a channel (7) provided in the wall of said dental device (1), said channel (7) comprising a cavity (6) defined within the wall of said dental device (1) and at least one opening (4, 5) connecting said cavity (6) to the exterior.

The present invention is also related to a method of manufacturing said dental device (1) by additive manufacturing and to methods for circulating a substance and/or saliva and oxygen through the dental device (1) in view of releasing said substance and/or saliva and oxygen inside the mouth.

## Description

### FIELD OF THE INVENTION

The present invention is related to a dental device used to cover at least part of the teeth, gums and other parts of the mouth such as an aligner tray, a retainer tray, a mouthguard, a nightguard, and similar. Said device has integrated micro or macro-fluidic channels and reservoirs which can contain one or multiple beneficial substances such as antibacterial liquid, fluoride, vitamins, flavor or other substances.

The present invention is also related to a method for manufacturing said dental device and to methods for circulating a substance and/or saliva and oxygen through the dental device.

### BACKGROUND OF THE INVENTION

Clear aligners as disclosed for example in document US 2022/0374119 A1 play a significant role in the dental industry, particularly in orthodontics and the treatment of teeth misalignment. They are advanced products that are almost invisible and are used for moderate or mild correction of orthodontic problems. As an alternative to traditional metal braces, clear aligners provide patients with a discreet and comfortable option for straightening their teeth. Usually, a set of 7-10 aligners is manufactured, each one being unique and contributing to an incremental movement of the teeth from the initial position to the final desired state. Each aligner is worn for about 2 weeks and is then replaced with the subsequent aligner in the series. Classical aligners are made by 3D printing molds which represent the negative of the aligner, and then thermoforming sheet of polymer on top of them. Afterwards, the set of aligners is cut out from the sheet and postprocessed. This is somewhat limiting the geometry and the precision of the manufactured aligner since supplementary features are difficult to add.

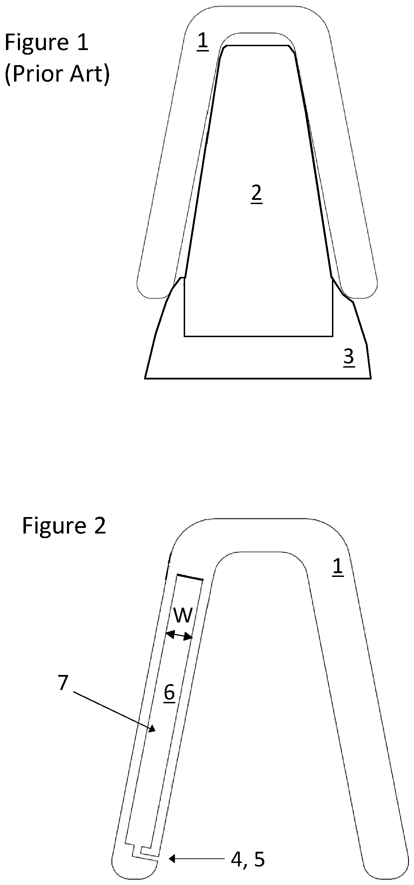

One of the limitations of conventional aligners 1 as shown in Figure 1 is well known. Due to the geometry of the aligner 1, the saliva and oxygen paths are obstructed and they cannot reach the teeth 2 or at least part of the gums 3. Saliva contains proteins, carbohydrates and immunoglobulins that interfere with bacterial metabolism and bacterial adherence to oral surface. Saliva is as well a solvent which controls the mouth odor. Thus, the reduced oxygen concentration and saliva content under the dental aligning device leads to increased bacteria buildup on the surface of patient's teeth as well as on the aligner itself. Since they are worn for prolonged periods of time, their close contact with the teeth can lead to detrimental side effects like tooth decay, dental plaque accumulation, gums disease, odor and bad breath as well as other oral infections like abscesses. Since clear aligners are most often made out of polyurethane, it is not recommended by the manufacturers to clean or sterilize them under high temperature by the end-user, which makes their maintenance more difficult.

Attempts have been made to mitigate this problem, but they were mostly limited to adding an additional layer, foam or gel to the classical thermoformed aligner as described in documents EP 1318761 B1 and WO 2018/018098 A1.

Although such an approach can lead to a certain improvement, it has a limited duration since the gel/foam are directly placed on the aligner and are worn of in a short period of time. As well, they can have detrimental effects on the aligning function of the device, since they might hinder proper adherence of the device to the teeth or reduce the overall applied force.

### SUMMARY OF THE INVENTION

The present invention aims to provide a dental device overcoming the drawbacks of the prior art. To this end, the dental device according to the invention comprises at least one channel provided in the wall of said dental device. Said channel comprises a cavity defined within the wall of said dental device and at least one opening connecting said cavity to the exterior. The expression "channel" is used here as a generic term and should not be interpreted as a geometric limitation implying any type of shape or ratio concerning the dimensions of the cavity or the opening.

The channel may advantageously be coated or preferably filled, or partially filled with a beneficial fluid, such as an antibacterial agent, specific medication, fluoride, remineralization agents, vitamins, flavour or other substances. The channel may be especially designed to act as a fluid reservoir that slowly releases said beneficial fluid onto the surface of the teeth or gums or more generally in the mouth during the use of the aligner.

The channel may advantageously comprise two or more openings facilitating the circulation of liquids and/or air through the dental device. In this case, the channel may only act as a saliva and oxygen pathway to the teeth and gums, the provision of an added beneficial fluid being optional.

More specifically, the present invention is related to a dental device intended to cover at least part of a teeth and optionally at least part of gums or other surfaces of a mouth for repositioning, retaining, protecting and/or treating said teeth and optionally said gums or other surfaces of the mouth, said dental device comprising a channel provided in the wall of said dental device, said channel comprising a cavity defined within the wall of said dental device and at least one opening connecting said cavity to the exterior.

If a conventional aligner is simply coated on the surface, the active time of the coating is very limited. But by integrating fluidic channels in the aligner, this duration can be significantly increased, fine-tuned and even customized per customer requirements. Additionally, multiple channel paths with various inward/outward facing geometries can be designed to serve different functions. These include various drugs/medications, antibacterials, fluoride for enhanced teeth remineralization, vitamins, flavor releasing channels, simple oxygen/saliva pathways or others. Various substances can be used to treat different dental issues, for example:
1) Drugs and antibacterial liquid would be used to treat gums inflammation, reduce bacteria buildup, reduce risk of tooth decay, and treat other problems.
2) Fluoride could be added to the same or a parallel channel in order to increase the strength of the tooth enamel through remineralization. This could lead to a novel approach in tooth remineralization.
3) Flavor would be used to remove the mouth odor and bad breath. The openings of these channels would preferably be facing the outer surface of the dental device and not the teeth. This would lead to significantly improved customer experience during the utilization of the dental device. These channels could be on purpose designed by adapting the size, shape, location and number of openings, such that when drinking water, it also passes through them and releases the flavor.
4) Channels would also be used to pass saliva and oxygen to the teeth. The ability of the microfluidic channels to pass oxygen and saliva to the teeth not only improves the patient's oral hygiene but may also require less frequent cleaning of the aligner, which would lead to enhanced customer convenience. Less frequent aligner removal would also reduce the wear on the aligner and can increase its lifetime and effectiveness.

According to particular embodiments of the invention, the dental device has one or several of the following features:
- the channel comprises two or more openings ;
- the dental device comprises several channels provided in the wall of said dental device, which are not in fluidic communication, each of said channels comprising a cavity defined within the wall of said dental device and at least one opening connecting said cavity to the exterior;
- at least two channels are disposed with at least one opening of one channel configured to be oriented toward the mouth in opposite direction to the teeth and with at least one opening of another channel configured to be oriented toward the teeth ;
- said at least one opening forms a connector or said at least one opening is provided with an external sacrificial connector to be removed after cleaning or filling, said connector or external sacrificial connector being designed to introduce a tool for cleaning the channel ;
- the dental device comprises a reinforcement disposed in the channel
- the reinforcement comprises a permeable lattice or a structure with one or several semi-permeable walls, meshes or openings ;
- the dental device comprises a one-way valve, cap or lid covering the at least one opening ;
- the dental device comprises a plug or film covering the at least one opening, to be removed before or during use preferably by dissolution in saliva ;
- the dental device is made up of polymer, preferably a shape memory polymer.

The invention also relates to a kit comprising the dental device and at least one plug or film intended to cover the at least one opening. Indeed the dental device and the plug or film could be provided separately.

Other dental applications can also benefit from such targeted delivery of medications and other substances where this is required over a prolonged period of time. It should be noted that said device is not limited only to aligners, but to the whole family of similar products which are used to cover the teeth such as retainers, a mouthguard, a nightguard, and similar devices.

The dental device according to the invention can be advantageously manufactured by 3D printing, for example, by techniques such as SLA *(Stereolithography),* DLP *(Digital Light Processing)* and FDM *(Fused Deposition Modelling)* techniques. With the recent advancements in the field of additive manufacturing, it is becoming possible to use a wide range of novel materials to manufacture parts with increasingly finer resolution and complex geometries in short times like the dental device according to the invention.

More specifically, the invention relates to a method for manufacturing the dental device as aforementioned, the method comprising a step of manufacturing the dental device with the channel provided in the wall of said dental device by additive manufacturing, said at least one opening of the channel being manufactured during this step or during a further step of micro-machining.

According to a particular embodiment of the method, the dental device comprises several openings provided in one channel or several channels, at least one of the several openings being manufactured during the further step of micro-machining.

The invention also relates to methods for circulating a substance and/or saliva and oxygen through the dental device in view of releasing said substance and/or saliva and oxygen inside the mouth.

More specifically, the invention relates to a method for circulating a substance in the dental device mounted inside the mouth for covering at least part of the teeth and optionally at least part of gums or other surfaces of the mouth, the method comprising before use a step of filling at least partially the cavity with the substance via the at least one opening and a step of releasing said substance inside the mouth through the at least one opening.

More specifically, the invention relates to a method for circulating saliva and oxygen in the dental device mounted inside the mouth for covering at least part of the teeth and optionally at least part of gums or other surfaces of the mouth, said dental device comprising at least one opening acting as a channel inlet oriented in opposite direction to the teeth and at least one opening acting as a channel outlet oriented toward the teeth, said saliva and oxygen circulating between the channel inlet and the channel outlet to be released in the direction of the teeth.

### BRIEF DESCRIPTION OF THE FIGURES

For sake of clarity, the teeth and the gums have not been represented in all figures.
Figure 1 represents a cross-sectional view of a conventional dental device of the prior art.
Figure 2 represents a cross-sectional view of a dental device with an integrated channel according to the invention.
Figures 3 to 8 represent cross-sectional views of a dental device with an integrated channel according to other variants of the invention.
Figure 9 represents three-dimensional views of a lattice structure which can be used as reinforcement in the dental device of Figure 8.
Figures 10 and 11 represent cross-sectional views of a dental device with an integrated channel according to other variants of the invention with said dental device covering the teeth and the gums.
Figure 12 represents a cross-sectional view of a dental device with an integrated channel according to another variant of the invention including a sacrificial connector on a channel opening acting as an inlet.
Figure 13 represents a cross-sectional view of a dental device with an integrated channel according to another variant of the invention including an integrated one-way valve on a channel opening acting as an inlet and plugs and films on channel openings acting as outlets.

### DESCRIPTION OF THE INVENTION

The dental device according to the invention is designed to cover at least part of the teeth and optionally at least part of the gums and/or other surfaces of the mouth for repositioning, retaining, protecting and/or treating said teeth and optionally said gums and/or other surfaces of the mouth.

The dental device 1 comprises at least one channel 7 comprising a cavity 6 defined within the wall of the dental device 1, and at least one opening 4, 5 connecting said inner cavity 6 with the exterior (see for example Figure 2). The cavity 6 may act as a reservoir for a beneficial fluid. The opening 4, 5 can be used as an inlet to introduce said beneficial fluid in the cavity 6. The opening 4, 5 may also allow a slow release of the fluid into the mouth when the dental device is worn by a user. A same opening can be used as inlet and outlet or at least two distinct openings can respectively be used as inlet, also called channel inlet with the reference number 4, and as outlet, also called channel outlet with the reference number 5.

The width W of the channel cavity 6 is limited by the width of the wall of the dental device 1, which is typically in the order of 1 mm, but can be also made thicker (2 or 3 mm) as long as it does not become too cumbersome to wear for a user. Typically, the cavity width will be lower than 1 mm, preferably between 50 and 500 micrometers. The dental device can comprise one, two, three, four or more channels which are not in fluidic communication, with each channel delivering a different substance or a same substance for a better control and localized delivery of the substance as illustrated hereafter. For each channel, the dental device can comprise one, two, three, four or more openings.

The size and number of the openings can be adapted to the nature of the beneficial fluid and to the intended use of the dental device. If the openings are too large and the beneficial fluid exhibits a relatively low viscosity and / or low surface tension, it may pour too rapidly out of the reservoir. If on the other hand the openings are too small, or too few, the circulation of the beneficial fluid, air or saliva may be hindered and be ineffective. Typically, the diameter of the openings with a circular cross section will be below 1 mm and can be as low as a few tens of micrometers. The openings can be provided as a plurality of micro-pores at the wall of the dental device, in communication with the cavity.

The openings can be configured to face the teeth, the gums or any other desired part of the mouth, the orientation of the openings being dependent on their role. One or more openings can form connectors to introduce a syringe or similar device, also called tool. The connector facilitates the rinsing and cleaning of the channel after production by connecting the syringe or similar device. As well, the connector can be used as an inlet for loading a substance in the cavity. The connector can be integrated by design to the dental device and form an elongated hole provided through the dental device to accommodate the syringe or similar device. In a variant, it can be a sacrificial connector connected at the channel opening and monolithically manufactured by 3D printing with the dental device. Such a sacrificial connector can be easily removed after playing its role of cleaner or inlet by cutting it off from the main body of the aligner along a designed cut-off line.

Integrated elastic one-way valves, caps or lids at openings acting as channel inlet or outlet can be designed and manufactured. These valves allow the beneficial liquid to be poured in the channels during the manufacturing of the dental device, but afterwards prevent the release of the liquid through the channel inlets. In addition, or in a different variant, plugs or films can be used to temporarily plug the channel inlets or the channel outlets, and to be removed before use. These plugs and films can be dissolvable in saliva, and thus be dissolved briefly upon the start of the dental device's use.

Before use, the substance fills at least partially the channel and could possibly only form a coating on the walls of the channel. In a variant, the channel is not filled with any substance before use, said channel being provided for the circulation of the saliva and oxygen to the teeth.

To increase the rigidity of the aligner or other similar dental device, reinforcement can be provided inside the channel. This allows to increase the ratio between the width of the channel and the thickness of the wall of the dental device without reducing the rigidity and mechanical properties of the device. The reinforcement can adopt different shapes. It may comprise one or more reinforcement beams across the cavity, provided at key positions where the device wall is expected to be exposed to significant mechanical stress. Alternatively, or in addition, the reinforcement may comprise a permeable lattice within the cavity, defined for instance as an open-cell structure. Further geometries such as reinforcement walls with holes, meshes, and others might be used as well.

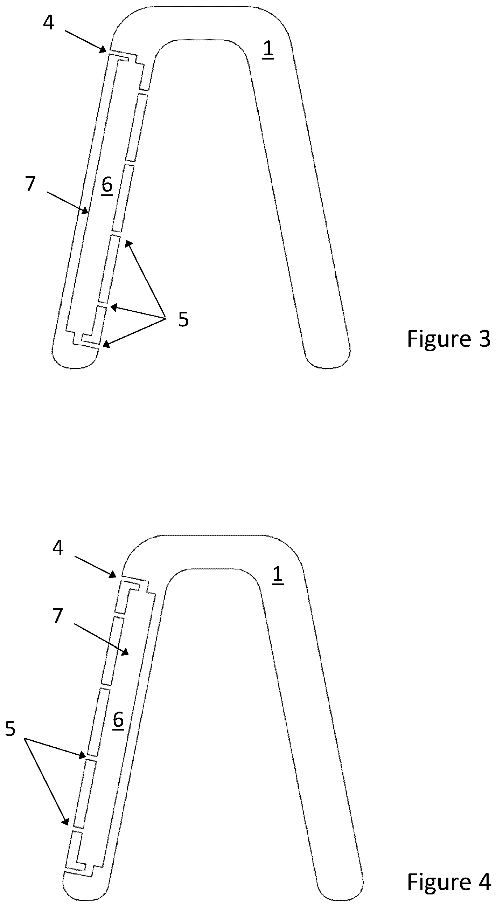

The dental device is made up of polymeric material and can be manufactured by any techniques of 3D printing such as for example SLA, DLP and FDM techniques. For example, the polymeric material can be a biocompatible photocurable resin such as the commercially available Biomed Clear (Formlabs), Figure 4^{™} MED-AMB 10 (3D Systems), KeySplint Soft Classic (KeyStone), Dental Clear PRO (Harz Labs), DSG (LuxCreo) or others. The aligner could also be manufactured out of a rubber. In a preferred embodiment, said dental device is manufactured out of a shape memory polymer like the commercially available TC-85DAC (Graphy), Biocompatible Shape Memory resin (3Dresyns), Clear (ODS) or others. In this last case, the polymer can be activated at a predefined temperature so that the dental device retrieves its initial shape. This is advantageous since these devices may lose their original shape by normal wear or accidentally as a result of inadequate manipulation.

Regardless of the material, a 3D printing process can be used to create a device of the invention directly comprising the openings of the channels, defined in the 3D-printed design file. This has the advantage of the simplicity of a one-step fabrication process.

Alternatively, or in addition, some or all of the openings can be realized in a subsequent step of micro-machining. By micromachining we refer to any known technique suitable for producing small holes in the wall of the dental device, including for example: piercing, drilling or laser ablation. This approach has the advantage of enabling better control of the size and shape of micro-metric holes than could be achieved with the limited resolution of some 3D-printing machines.

In a preferred embodiment, said device is transparent in color, but it could also be manufactured in various colors for esthetical purposes. Similarly, the substances introduced in the reservoirs can be transparent or colored depending on the esthetical requirements.

Different embodiments of the dental device are represented in Figures 2 to 13.

In Figure 2, an embodiment of the dental device 1 with one integrated channel 7 is represented. The integrated channel 7 forms the reservoir 6 with a single opening 4, 5, which can be first used as inlet for filling the reservoir 6 with a beneficial substance, and then, during use, as outlet to deliver the beneficial substance to the teeth. In this example, the opening is defined on the side of the device wall which faces the teeth and gums when the device is worn. The beneficial fluid with a possible medical effect can be locally released in this region, the release being driven by simple diffusion through the opening 4,5.

In the example of Figure 3, the dental device 1 also comprises a single channel 7 provided with a cavity 6 defined within the wall of the dental device 1. However, in this case, several openings 4, 5 communicate the cavity 6 with the exterior. In this example, the opening 4 can be used as an inlet to introduce a beneficial fluid in the cavity 6, whereas the openings 5, facing the teeth when the device is worn, can act as outlets releasing the beneficial fluid at the interface with the teeth and gums.

The device of Figure 3 may also be used without the addition of any substance. The channel 7 can be beneficial simply by allowing the circulation of air and saliva from the outer side of the dental device 1 to the inner side in contact with the teeth. For this application, it can be advantageous to provide additional openings 4 (not represented) on the outer surface of the dental device 1 to enhance the circulation of fluids. In another embodiment as shown in Figure 4, the channel outlets 5 are facing the mouth in the opposite direction to the teeth. In such an embodiment the integrated channel would most likely be used for flavor containing substances.

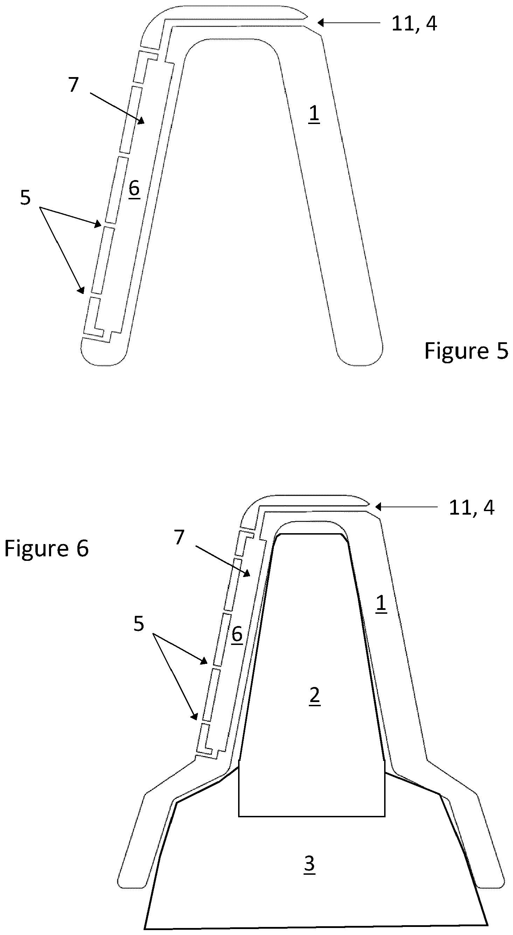

In another embodiment as shown in Figure 5, the channel inlet 4 is integrated by design to the dental device 1 to form a connector 11. This will facilitate the rinsing and cleaning of the integrated channel after production by connecting syringes and similar devices. As well, this integrated inlet is used for loading the substance in the reservoir.

Figure 6 shows a different embodiment where the dental device 1 covers at least some of the teeth 2 but also at least part or all of the gums 3 and other surfaces in the mouth. Such a dental device can be used to selectively bring medication or other beneficial substances to gums 3 or other parts of the mouth in order to treat other dental issues. Such a design of the dental device covering at least part of the gums can as well be applied to all the other embodiments of the invention.

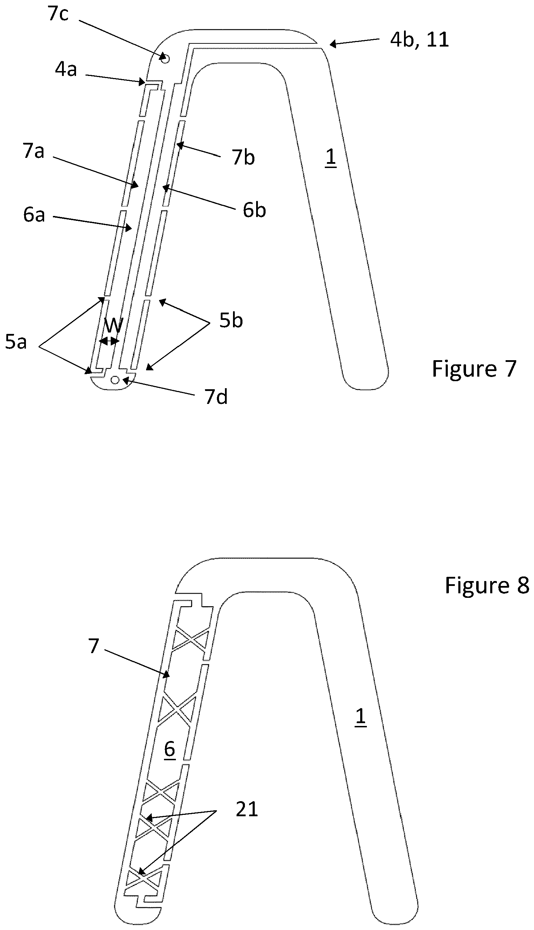

In another embodiment as shown in Figure 7, the dental device 1 has multiple integrated channels 7a, 7b, 7c, 7d which are not in fluidic communication. These channels are designed to contain different beneficial substances, or for a better control and localized delivery of the same substance. For example, a first channel 7a has an inlet 4a, the reservoir 6a and mouth-facing outlets 5a, which would most likely be used for flavor containing substance. The second channel 7b has an inlet 4b forming a connector 11, the reservoir 6b and outlets 5b which are facing the teeth. This channel would most likely be used for antibacterial liquid, drugs, or fluoride. Other channels might be also included, such as the third and fourth channels 7c, 7d which are seen just as a tubes in this cross section.

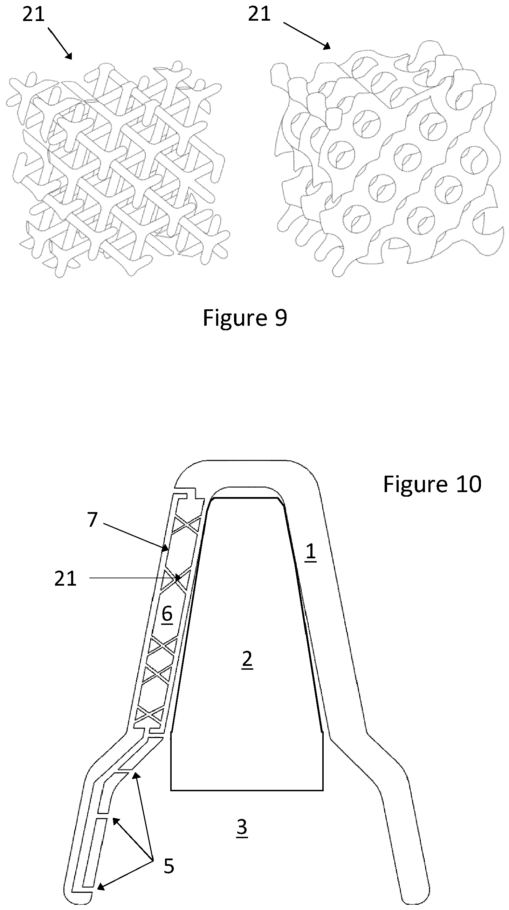

To increase the rigidity of the aligner or other similar dental device, a reinforcement 21 can be defined within the channel 7 as shown in Figure 8. The reinforcement 21 may comprise one or more reinforcement beams across the cavity 6, provided at key positions where the device wall is expected to be exposed to significant mechanical stress. Alternatively, or in addition, the reinforcement may comprise a permeable lattice within the cavity, defined for instance as an open-cell structure. Some examples of such open-cell structures which can be implemented as permeable reinforcement lattices 21 are shown in Figure 9. The reinforcement structures 21, whether defined as beams or lattices, may be advantageously fabricated simultaneously and by means of the same additive manufacturing process as the rest of the dental device 1.

In another embodiment shown in Figure 10, a dental device 1 with an integrated channel 7 and a reinforcement 21 is also covering at least part of the gums 3 and has channels outlets 5 facing zones of interest on the gums 3. Conveniently, the part of the dental device 1 in direct contact with the teeth 2 is provided with a reinforcement structure 21. This confers a degree of rigidity to the structure which may be needed to apply pressure on the teeth as required in orthodontic treatments. In contrast, the part of the dental device 1 in direct contact with the gums 3 can be designed with low rigidity, to reduce and distribute the pressure on the gums 3, increasing the comfort for the user and reducing the risk of damaging these sensitive parts.

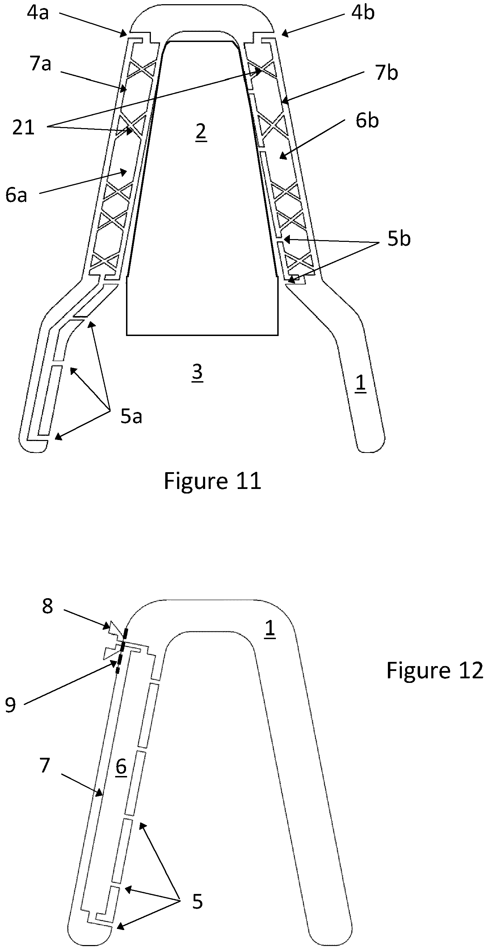

In another embodiment shown in Figure 11, a dental device 1, covering teeth 2 and gums 3 on both sides has two channels 7a, 7b with respective inlets 4a, 4b, and reservoirs 6a, 6b. In this embodiment, reinforcements 21 with, for example, permeable lattices are used to reinforce the rigidity of the dental device 1. Integrated channels 7a, 7b and their inlets 4a, 4b are located on the opposite sides of the teeth 2, and in this particular case, the reservoir 6a of the channel 7a delivers beneficial substances to the gums 3 via outlets 5a, while the reservoir 6b of the channel 7b is oriented towards the teeth 2 via outlets 5b.

In order to fully benefit from the fact that the increase in complexity in additive manufacturing comes almost for free, but can bring a significant added value, in another embodiment shown in Figure 12, a sacrificial connector 8 is added to the dental device 1. This has the same role as the integrated connector 11 described in Figure 5. The advantage of the sacrificial connector is that after the dental device has been cleaned and filled up with a beneficial substance, it can be easily removed by cutting/breaking it off along a cut line 9, thus leaving the outer surface compact as compared to the integrated connector 11 presented in Figure 5. The cut line 9 can be defined by zones which have been weakened by design on purpose, thus facilitating the removal of connectors 8.

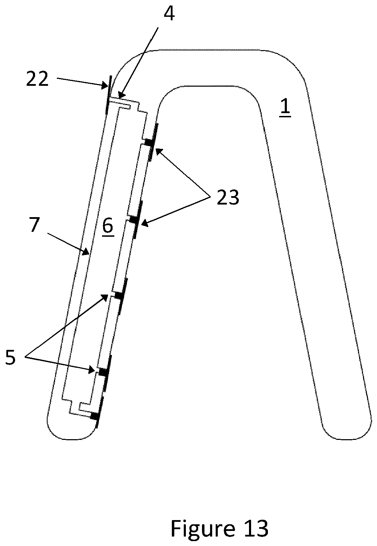

In another embodiment, as shown in Figure 13, an integrated elastic one way valve, cap or lid 22 on the channel inlet 4 is designed and manufactured. This valve allows the beneficial liquid to be poured in the channel during the manufacturing of the dental device, but afterwards prevents the liquid to be released - namely through the channel inlet. Plugs or films 23 can be used to temporarily plug the channel outlets 5, and to be removed before use. These plugs and films can be also dissolvable in saliva, and thus be dissolved briefly upon the start of the dental device's use.

All figures are shown as preferred embodiments but should not be taken as limiting factors and various other designs could be envisaged. The embodiments of the invention and materials suggested for the device in the present application are only illustrative examples and should not be construed in a limiting manner. The present invention may also use equivalent means and other geometries or other manufacturing methods which would allow to achieve similar results as the ones described herein with corresponding results. The embodiments described herein may also be combined together.

## Claims

1. A dental device (1) intended to cover at least part of a teeth (2) and optionally at least part of gums (3) or other surfaces of a mouth for repositioning, retaining, protecting and/or treating said teeth (2) and optionally said gums (3) or other surfaces of the mouth, said dental device (1) comprising a channel (7) provided in the wall of said dental device (1), said channel (7) comprising a cavity (6) defined within the wall of said dental device (1) and at least one opening (4, 5) connecting said cavity (6) to the exterior.

2. The dental device (1) as in claim 1, wherein the channel (7) comprises two or more openings (4, 5).

3. The dental device (1) as in any of previous claims, wherein the dental device (1) comprises several channels (7a, 7b, 7c, 7d) provided in the wall of said dental device (1), which are not in fluidic communication, each of said channels (7a, 7b, 7c, 7d) comprising a cavity (6a, 6b) defined within the wall of said dental device (1) and at least one opening (4a, 4b, 5a, 5b) connecting said cavity (6a, 6b) to the exterior.

4. The dental device (1) as in claim 3, wherein at least two channels (7a, 7b) are disposed with at least one opening (5a, 5b) of one channel (7a, 7b) configured to be oriented toward the mouth in opposite direction to the teeth (2) and with at least one opening (5b, 5a) of another channel (7b, 7a) configured to be oriented toward the teeth (2).

5. The dental device (1) as in any of previous claims, wherein said at least one opening (4,5) forms a connector (11) or wherein said at least one opening (4,5) is provided with an external sacrificial connector (8) to be removed after cleaning or filling, said connector (11) or external sacrificial connector (8) being designed to introduce a tool for cleaning the channel (7).

6. The dental device (1) as in any of the previous claims, wherein said dental device (1) comprises a reinforcement (21) disposed in the channel (7).

7. The dental device (1) as in claim 6, wherein the reinforcement (21) comprises a permeable lattice or a structure with one or several semi-permeable walls, meshes or openings.

8. The dental device (1) as in any of the previous claims, wherein said dental device (1) comprises a one-way valve, cap or lid (22) covering the at least one opening (4, 5).

9. The dental device (1) as in any of the previous claims, wherein said dental device (1) comprises a plug or film (23) covering the at least one opening (4, 5), to be removed before or during use preferably by dissolution in saliva.

10. The dental device (1) as in any of the previous claims, wherein the dental device (1) is made up of polymer, preferably a shape memory polymer.

11. The dental device (1) as in any of the previous claims, wherein said dental device (1) is an aligner, a retainer, a mouthguard or a nightguard.

12. A method for manufacturing the dental device (1) according to any of the previous claims, comprising a step of manufacturing the dental device (1) with the channel (7) provided in the wall of said dental device (1) by additive manufacturing, said at least one opening (4, 5) of the channel (7) being manufactured during this step or during a further step of micro-machining.

13. The method as in claim 12, wherein the dental device (1) comprises several openings (4,5) provided in one channel (7) or several channels (7a, 7b, 7c, 7d), at least one of the several openings (4, 5) being manufactured during the further step of micro-machining.

14. A method for circulating a substance in the dental device (1) according to any of claims 1 to 11, with said dental device (1) mounted inside the mouth for covering at least part of the teeth (2) and optionally at least part of gums (3) or other surfaces of the mouth, the method comprising before use a step of filling at least partially the cavity (6) with the substance via the at least one opening (4,5) and a step of releasing said substance inside the mouth through the at least one opening (4,5).

15. A method for circulating saliva and oxygen in the dental device (1) according to any of claims 2 to 11, with said dental device (1) mounted inside the mouth for covering at least part of the teeth (2) and optionally at least part of gums (3) or other surfaces of the mouth, said dental device (1) comprising at least one opening (4,5) acting as a channel inlet (4) oriented in opposite direction to the teeth (2) and at least one opening (4,5) acting as a channel outlet (5) oriented toward the teeth (2), said saliva and oxygen circulating between the channel inlet (4) and the channel outlet (5) to be released in the direction of the teeth.
